# EUROPÄISCHE PATENTSCHRIFT

(11) **EP 0 880 366 B1**
(45) Veröffentlichungstag und Bekanntmachung des Hinweises auf die Patenterteilung: **12.09.2001**
(21) Anmeldenummer: 96944648.3
(22) Anmeldetag: 23.12.1996
(51) Int. Cl.: A61L 15/44

(54) **HAUTHAFTENDE PHARMAZEUTISCHE ZUBEREITUNG, INSBESONDERE TTS ZUR ABGABE VON 17-BETA-ESTRADIOL AN DEN MENSCHLICHEN ORGANISMUS**
SKIN-ADHERING PHARMACEUTICAL PREPARATION, IN PARTICULAR TRANSDERMAL THERAPEUTIC SYSTEM FOR THE RELEASE OF 17-BETA-ESTRADIOL TO THE HUMAN ORGANISM
PREPARATION PHARMACEUTIQUE ADHERANT A LA PEAU, NOTAMMENT SYSTEME THERAPEUTIQUE TRANSDERMIQUE POUR LA LIBERATION DE 17-BETA-ESTRADIOL DANS L'ORGANISME HUMAIN

(30) Priorität: 08.01.1996 DE 19600347
(43) Veröffentlichungstag der Anmeldung: 02.12.1998
(73) Patentinhaber: LTS Lohmann Therapie-Systeme AG, 56626 Andernach (DE)
(72) Erfinder: ASMUSSEN, Bodo, D-56170 Bendorf (DE); HORSTMANN, Michael, D-56564 Neuwied (DE)
(74) Vertreter: Flaccus, Rolf-Dieter, Dr.
(86) Internationale Anmeldenummer: EP9605822
(87) Internationale Veröffentlichungsnummer: WO9725077

(56) Entgegenhaltungen:
- EP-A- 0 413 034
- WO-A-96/05814
- DE-A- 4 405 899
- DE-C- 4 223 360
- DE-C- 4 237 453

## Beschreibung

Die Erfindung betrifft eine hauthaftende pharmazeutische Zubereitung, insbesondere ein transdermales therapeutisches System, zur Abgabe von 17-β-Estradiol und gegebenenfalls weiteren Wirkstoffe durch die Haut an den menschlichen Organismus.

Pharmazeutische Zubereitungen mit Hautkontakt sind beispielsweise Salben, Cremes, Lotionen oder auch arzneistoffhaltige Pflaster, die unter der Bezeichnung "transdermale therapeutische Systeme" (TTS) für eine Therapierung einer Reihe von Erkrankungen im Markt eingeführt sind.
Auch TTS mit Wirkstoff 17-β-Estradiol sind inzwischen als Therapeutikum für Wechseljahrbeschwerden, neuerdings auch gegen Osteoporose, im Handel und bewähren sich erfolgreich in der Therapie.

Als Nachteil dem Stand der Technik entsprechender Systeme werden in einer Reihe von Fällen eine nicht ausreichende Permeationsfähigkeit des Wirkstoffes durch die Haut beobachtet, die auch durch zahlreiche galenische Maßnahmen des TTS-Designs (Einsatz mehrschichtiger Systeme, Verwendung von Steuermembranen, Variationen der Wirkstoffkonzentration, Modifikation der Grundpolymeren etc.) nicht über eine gewisse Grenze, den sogenannten "Sättigungsfluß", hinaus steigerbar ist.

Die Feststellung, daß der transdermale Fluß eines Wirkstoffes aus der festen, fein verteilten Phase heraus auch bei Einsatz stärker lösender Vehikel nicht weiter steigerbar ist, findet sich bereits in den wegweisenden Arbeiten von Higuchi (z.B. T.Higuchi: Physical Chemical Analysis of percutaneous absorption process from creams and ointments, J. Soc.Cosmetic Chem. 11, S.85-97 (1960)). Allerdings gibt es für viele Wirkstoffe die Möglichkeit, dem TTS bei der Herstellung sogenannte "Enhancer" zuzusetzen. Es handelt sich hierbei in der Regel um flüssige Zusatzstoffe, die die Resorptionseigenschaften der menschlichen Haut verbessern und damit die Aufnahme des Wirkstoffes aus einer genügend kleinen TTS-Fläche heraus ermöglichen.

Speziell leicht flüchtige Enhancer wie das beispielsweise für den Wirkstoff 17-β-Estradiol vielfach verwendete Ethanol ergeben Probleme infolge starker Erweichung von Klebschichten der Pflaster und erfordern dadurch raumfordernde Kompartimente im System, die das TTS unakzeptabel dick oder umfangreich werden lassen. Schließlich birgt auch jeder weitere nichtpolymere Zusatzstoff die Gefahr von Unverträglichkeiten auf der Haut, unter Umständen auch von Sensibilisierungen.
Unter Zusatz weniger flüchtiger, meist aber auch weniger aktiver Enhancer (z.B. Glycerinester, cyclische Amide, Eucalyptol) ist zwar die Herstellung von Matrixsystemen möglich, die Wirkstoff und resorptionsverstärkende Komponenten in einer oder mehreren monolithischen Schichten enthalten. Die Klebkraft des Pflasters bleibt jedoch unbefriedrigend.
Das Dokument US 4 863 738 stellt ein Beispiel von vielen dar, in denen die Applikation eines Wirkstoffes, z.B. 17-β-Estradiol, zusammen mit einem Enhancer (hier Glycerinmonooleat) in einer TTS-Matrix in beliebiger Konzentration beansprucht wird.
Nach dem Stand der Technik ist jedoch mit solchen TTS keine befriedigende Therapie möglich, da die ausgewählten Enhancer entweder zu schlecht hautverträglich sind, oder die Systeme wegen zu geringen Wirkstoffflusses durch die Haut unakzeptabel große Flächen aufweisen müssen.

Eine andere Möglichkeit, den Wirkstofffluß durch die Haut zu steigern, könnte darin bestehen, mehr Wirkstoff im TTS molekulardispers aufzulösen als dies der Sättigungslöslichkeit entspricht. Mit dem Ausmaß der Übersättigung solcher Systeme wird im gleichen Maße auch die Permeationsgeschwindigkeit durch die Haut gesteigert. Da übersättigte physikalische Zustände thermodynamisch jedoch instabil sind, erweisen sich solche Arzneiformen als nicht lagerfähig. Es finden innerhalb von Monaten und spätestens Jahren spontane, unvorhersehbare Ausfällungen von Wirkstoffteilchen statt, so daß die Flußrate durch die Haut nach und nach auf das Sättigungsflußniveau abfällt und damit ein großer Teil der anfangs vorhandenen therapeutischen Aktivität verloren geht.

Die in EP 0 421 454 beschriebenen Systeme enthalten 17-β-Estradiol in einem Acrylatpolymer unter Zusatz von "Kristallisationshemmern" und klebrigmachenden Harzen. Quellstoffe sind zum Schutz vor vorzeitigem Klebkraftverlust enthalten.

Ein grundsätzlich anderer Weg zur Vermeidung thermodynamischer Instabilität wurde mit der DE 42 37 453 beschritten. Darin ist ein transdermales therapeutisches System beschrieben, dessen Wirkstoffkonzentration zwischen der Sättigungslöslichkeit unter feuchten Bedingungen und unter trockenen Bedingungen liegt. Unter "trockenen" Bedingungen wird eine relative Umgebungsfeuchte von höchstens 10% verstanden, und die erzielte Steigerung der Hautpermeation ist mit etwa 50 % angegeben.

Aufgabe der vorliegenden Erfindung ist es, eine pharmazeutische Zubereitung anzugeben, welche relativ zum Stand der Technik höhere Wirkstoffflüsse durch die Haut ergibt und während der Lagerung keine Aktivitätsverluste durch Rekristallisation bzw. Wirkstoffausfällung erleidet.

Diese Aufgabe wird erfindungsgemäß bei einer pharmazeutischen Zubereitung entsprechend dem Oberbegriff von Anspruch 1 durch die aus den kennzeichnenden Merkmalen des Anspruchs 1 entnehmbaren Merkmale gelöst, indem die in der Zubereitung enthaltene Estradiolmenge mindestens das Dreifache der bei 95% Luftfeuchtigkeit gemessenen Sättigungslöslichkeitsmenge beträgt und die im Packmittel vorhandene Luft auf eine relative Luftfeuchtigkeit von unter 5%, bevorzugt unter 2% relativer Luftfeuchtigkeit, eingestellt ist.

17-β-Estradiol liegt bei Raumtemperatur und relativer Luftfeuchtigkeit zwischen 20 und 60% nicht in einer kristallwasserfreien Modifikation (I und II) vor, sondern als Semihydrat (Busetti und Hospital, Acta Cryst.1972, B28,560). Wegen der schichtförmigen, über Wasserstoffbrücken stabilisierten Struktur und infolge der Diffusionsdichtigkeit des Kristallverbundes läßt sich das Hydrat kurzzeitig unzersetzt bis zu Temperaturen von 170 Grad Celsius erhitzen (Kuhnert-Brandstätter u. Winkler (1976), Scientia Pharmaceutica 44 (3), 177-190).
Anderseits weist 17-β-Estradiol mit geringer werdendem Wasserdampfpartialdruck höhere Löslichkeiten in einigen Polymeren, speziell in Acrylatcopolymeren, auf. Dies ist zwar bereits aus DE 42 37 453 bekannt, jedoch ist das Ausmaß der Löslichkeitssteigerung auf mehr als das Dreifache auch unter Zugrundelegung des Standes der Technik unerwartet und überraschend (Beispiel 1). Da mit höherer Konzentration des Wirkstoffs in einem TTS unter sonst gleichen Bedingungen der Diffusionsfluß durch die Haut nach dem Fickschen Gesetz zunimmt, ist eine solche Konzentrationssteigerung bei transdermalen therapeutischen Systemen sehr vorteilhaft. Dadurch können erfindungsgemäß hergestellte Zubereitungen bei kleinerer Fläche gleiche Wirksamkeit entfalten wie dem Stand der Technik entsprechende Systeme. Zur Stabilisierung dieser Eigenschaften sind jedoch absolut trockene Lagerbedingungen erforderlich.

Diese lassen sich bevorzugt durch gas- und feuchtigkeitsdichte Packmittel sowie Einlegen von feuchtigkeitsabsorbierenden Produkten in das Packmittel gewährleisten.

Die vorstehend beschriebene Erfindung kann auf unterschiedliche Weise in pharmazeutischen Zubereitungen realisiert werden.
In einer Salbe wird 17-β-Estradiol als Anhydrat oder als Semihydrat nach üblichen Verfahren durch Rühren unter Erwärmung und Zugabe einer Lösung von Estradiol in einem Lösemittel zur Salbengrundlage oder aber auch durch Dispergieren von mikronisiertem Wirkstoff homogen verteilt. Die einfachste Form einer perkutanen hauthaftenden pharmazeutischen Zubereitung ist ein einschichtiges Matrixsystem, dessen Matrix neben ihrer Funktion der Wirkstofflieferung gleichzeitig haftklebend ist und damit eine Klebschicht überflüssig macht; jedoch ist eine Aufteilung des Systems in mehrere Schichten gleicher oder unterschiedlicher Zusammensetzung und Funktion möglich. Wird zwischen einer solchen Matrix und einer hautseitig angeordneten Klebschicht eine für Estradiol schwer durchlässige Membran angebracht, erreicht man eine stärker vom Pflaster als von der Haut gesteuerte Wirkstoffabgabe. Diese kann vorteilhaft sein, wenn ein sehr enger Spielraum der täglichen Dosierung angestrebt wird.

Acrylsäurecopolymere sind in diesem Zusammenhang Copolymere, entstanden aus radikalischer Polymerisation von Estern der Acrylsäure und Methacrylsäure mit C₁ bis C₁₈-Alkoholen, Dimethylaminoethanol oder anderen geeigneten alkoholischen Reaktionspartnern, Vinylacetat, Vinylpyrrolidon, Styrol, Butadien, Acrylnitril oder anderen geeigneten Monomeren mit einer Vinylgruppe.

Zur weicheren Einstellung des Systems können zum Beispiel 1,2-Propandiol, 1,3-Butylenglycol, 1-Hexadecanol, zugesetzt werden oder 2-Hydroxyfettalkohole, 2-Octyldodecanol, 2-Propanol, Benzylalkohol, Cetylstearylalkohol, Diethylenglycol, Dipropylenglycol, Dodecanol, Ethanol, Glycerol, Hexandiol, Octanol, Oleylalkohol, Panthenol, Phenylethanol, Polyethylenglycole oder Polypropylenglycole bzw. Fettsäuren wie Carpin-, Linolein-, Laurin-, Myristinsäure, n-Valeriansäure, Pelargonsäure, auch physiologisch akzeptable organische Säuren wie 3-Phenylpropionsäure, Essigsäure, Adipinsäure, Benzoesäure, Ölsäure, Salicylsäure oder deren hautverträgliche Salze. Weitere Verbindungen dieser Art sind ohne Anspruch auf Vollständigkeit Sulfate und Sulfonate der Fettsäuren, Ester der Formel [CH₃(CH₂)ₘCOO]ₙR, wobei m eine Zahl von 8 bis 16, n 1 oder 2 und R eine kurzkettige Alkylkette darstellt, Triglyceride, Phthalate, Sulfoxide oder Amide.

Die Erfindung wird nachfolgend anhand von Beispielen näher erläutert.

### Beispiel 1: Herstellung eines erfindungsgemäßen Systems

3,6 g 17-β-Estradiol-semihydrat, mikronisiert, und 150,4 g Lösung eines Acrylatcopolymers mit 37,5 % Feststoffgehalt
werden bei Raumtemperatur in einem zylindrischen Glasgefäß bis zum Erreichen einer gleichmäßigen Suspension gerührt und anschließend auf 100 Mikrometer dicke, siliconisierte Polyesterfolie so beschichtet, daß eine Schichtdicke von 61 g/m² (bezogen auf den lösemittelfreien Anteil) resultiert. Daraus ergibt sich ein Anteil von 6 % (g/g) Estradiol in der wirkstoffhaltigen Schicht. Der Ausstrich wir 10 Minuten lang bei 25 Grad C und 15 Minuten bei 90 Grad C getrocknet, wobei sich unter dem Einfluß der Wärme der Wirkstoff vollständig auflöst. Anschließend wird 15 um starke Polyesterfolie als Rückschicht aufgelegt (zukaschiert).
Durch Stanzung mit Bandstahlwerkzeug werden transdermale Systeme von 16 cm² erhalten, die
a) bei 31°C/ 70 % relativer Feuchte
b) bei ca.20-30°C (Raumtemperatur), 0 % relativer Feuchte gelagert werden.

Nach dreiwöchiger Lagerung zeigen die Muster nach Bedingung a) (Stand der Technik) vollständige, makroskopisch erkennbare Kristallisationen. Muster der Bedingung b) (erfindungsgemäß) bleiben hingegen vollständig gelöst.

### Beispiel 2: Herstellung eines Systems(Vergleichsbeispiel)

0,9 g 17-β-Estradiol-semihydrat,mikronisiert, und 157,6 g Lösung eines Acrylatcopolymers mit 37,5 % Feststoffgehalt
werden bei Raumtemperatur in einem zylindrischen Glasgefäß bis zum Erreichen einer gleichmäßigen Lösung gerührt und anschließend auf 100 Mikrometer dicke, siliconisierte Polyesterfolie so beschichtet, daß eine Schichtdicke von 58 g/m² (bezogen auf den lösemittelfreien Anteil) resultiert.
Daraus ergibt sich ein Anteil von 1,5 % (g/g) frei gelösten Estradiols in der wirkstoffhaltigen Schicht.
Der ausstrich wird 10 Minuten lang bei 25 Grad C und 15 Minuten bei 90 Grad C getrocknet.
Anschließend wird 15 µm starke Polyesterfolie als Rückschicht aufgelegt (zukaschiert).
Durch Stanzung mit Bandstahlwerkzeug werden transdermale Systeme von 16 cm² erhalten, die
a) bei 31°C/70 % relativer Feuchte
b) bei ca. 20-30°C (Raumtemperatur),0 % relativer Feuchte gelagert werden.

Nach dreiwöchiger Lagerung zeigen die Muster nach Bedingung a) feine, mikroskopisch gut erkennbare Kristallisationen.
Muster nach Bedingung b) bleiben hingegen vollständig gelöst.

Die gleichmäßige Zufuhr von Estradiol hat sich aus zur Prävention der Alzheimerschen sowie der Parkinsonschen Erkrankung als wirksam erwiesen (Scrip No.2026, p.23, 19.Mai 1995). Eine ethisch besonders bedeutsame Anwendung der vorstehend beschriebenen hauthaftenden pharmazeutischen Zubereitungen liegt daher auch in dieser Indikation.

## Patentansprüche

1. Hauthaftende pharmazeutische Zubereitung, insbesondere TTS, zur Abgabe des Wirkstoffs 17-β-Estradiol, wobei die Konzentration des darin in gelöster Form enthaltenen Estradiols in allen Matrixschichten und bei Vorhandensein einer Klebschicht auch in dieser zwischen seiner Sättigungskonzentration im Gleichgewicht mit einer Gasphase mit weniger als 10 % relativer Luftfeuchtigkeit und seiner Sättigungskonzentration im Gleichgewicht mit einer Gasphase mit mehr als 90 % relativer Luftfeuchtigkeit steht, **dadurch gekennzeichnet, daß** die in der Zubereitung enthaltene Estradiolmenge mindestens das Dreifache der bei 95 % relativer Luftfeuchtigkeit gemessenen Sättigungslöslichkeit beträgt und die im Packmittel eingeschlossene Luft auf eine relative Luftfeuchtigkeit zwischen 5 % und unter 0,5 % eingestellt ist.

2. Zubereitung nach Anspruch 1, **dadurch gekennzeichnet, daß** die im Packmittel eingeschlossene Luft auf eine relative Luftfeuchtigkeit zwischen 5 % und unter 2 % eingestellt ist.

3. Zubereitung nach Anspruch 1, **dadurch gekennzeichnet, daß** die im Packmittel eingeschlossene Luft durch Zugabe eines Trockenmittels eingestellt ist.

4. Zubereitung nach Anspruch 1, **dadurch gekennzeichnet, daß** sie in transdermales therapeutisches System ist.

5. Zubereitung nach einem oder mehreren der vorhergehenden Ansprüche, **dadurch gekennzeichnet, daß** sie eine hautseitige haftklebende Klebschicht und eine nichtklebrige hautabgewandte Rückschicht aufweist.

6. Zubereitung nach einem oder mehreren der vorhergehenden Ansprüche, **dadurch gekennzeichnet, daß** sie ein Acrylsäureestercopolymer enthält.

7. Zubereitung nach Anspruch 6, **dadurch gekennzeichnet, daß** das Acrylsäureestercopolymer bei 0,1 % relativer Luftfeuchtigkeit mindestens 6 %, bevorzugt mindestens 8 % (g/g) 17-β-Estradiol in gelöster Form enthält.

8. Verfahren zur Herstellung primärverpackter hauthaftender pharmazeutischer Zubereitungen nach einem oder mehreren der vorhergehenden Ansprüche, **gekennzeichnet durch** Bereiten einer Lösung oder Dispersion von 17-β-Estradiol-Semihydrat in einer halbfesten oder flüssigen pharmazeutischen Grundlage und anschließendes Beschichten auf einem folienförmigen, dehäsiv ausgerüsteten Grundmaterial, Trocknen der Schicht und Auflegen einer wirkstoff- und feuchtigkeitsundurchlässigen Rückschicht und Vereinzelung durch Konturstanzen und Folienschnitt, **dadurch gekennzeichnet, daß** das Trocknen durch Zwischenlagerung bei Luftfeuchtigkeit von unter 5 %, bevorzugt unter 2 %, besonders bevorzugt unter 0,5 % relativer Feuchte, bis zur vollständigen Auflösung des enthaltenen Estradiols sowie anschließende Verpackung in gasdicht versiegelte Packmittel, gegebenenfalls unter Zugabe von feuchtigkeitsabsorbierendem Material, erfolgt.

9. Verwendung von Zubereitungen nach einem oder mehreren der Ansprüche 1 bis 7 zur Herstellung einer hauthaftender pharmazeutischen zurbereitung Behandlung von menopausalen Symptomen zur Osteoporoseprophylaxe oder zur Prophylaxe und Therapie der Alzheimerschen Erkrankung.

## Claims

1. A pharmaceutical preparation adhering to the skin, in particular a TTS, for the release of the active substance 17-β-estradiol, the concentration of the estradiol contained therein in dissolved form being between its saturation concentration in equilibrium with a gas phase of less than 10% relative air humidity and its saturation concentration in equilibrium with a gas phase of more than 90% relative air humidity, in all matrix layers and, if present, also in an adhesive layer, **characterized in that** the estradiol quantity contained in the. preparation amounts to at least three times the saturation solubility measured at, 95% relative air humidity, and that the air enclosed in the package is adjusted to a relative air humidity between 5% and below 0.5%.

2. The preparation according to claim 1 **characterized in that** the air enclosed in the package is adjusted to a relative air humidity between 5% and below 2%.

3. The preparation according to claim 1 **characterized in that** the air enclosed in the package is adjusted by the addition of a desiccant.

4. The preparation according to claim 1 **characterized in that** it is a transdermal therapeutic system.

5. The preparation according to one or several of the preceding claims **characterized in that** it has a pressure-sensitive adhesive layer facing the skin and a non-tack backing layer averted from the skin.

6. The preparation according to one or several of the preceding claims **characterized in that** it comprises an acrylic-acid ester copolymer.

7. The preparation according to claim 6 **characterized in that** the acrylic-acid ester copolymer at 0.1% relative air humidity comprises at least 6%, preferably at least 8% (w/w) 17-β-estradiol in dissolved form.

8. A process for the production of primary-packaged, skin-adherent pharmaceutical preparations according to one or several of the preceding claims, **characterized by** preparing a solution or dispersion of 17-β-estradiol-semihydrate in a semisolid or liquid pharmaceutical base followed by coating it onto a film-like, antiadhesive base material, drying the layer and applying a backing layer which is impermeable to active substances and moisture, and separating by blanking and film cutting, **characterized in that** drying is effected by intermediate storage at an air humidity of below 5%, preferably below 2%, and most preferably below 0.5% relative humidity, up to complete dissolution of the contained estradiol, and by packing into gastight packages, optionally with addition of a moisture-absorbing material.

9. The use of pharmaceutical preparations according to one or several of claims 1 to 7 for the production of a skin-adherent preparation intended for the treatment of menopausal symptoms, for osteoporosis prophylaxis, or for the prophylaxis and therapy of Alzheimer's disease.

## Revendications

1. Préparation pharmaceutique adhérant à la peau, en particulier un TTS, pour la distribution de la substance active 17-β-oestradiol, dans laquelle la concentration de l'oestradiol qui y est contenu sous forme dissoute dans toutes les couches matricielles et, en présence d'une couche adhésive, également dans cette dernière, entre sa concentration de saturation en équilibre avec une phase gazeuse dont la teneur en humidité relative de l'air est inférieure à 10 % et sa concentration de saturation en équilibre avec une phase gazeuse dont la teneur en humidité relative de l'air est supérieure à 90 %, **caractérisé en ce que** la quantité d'oestradiol contenu dans la préparation représente au moins le triple de la solubilité de saturation mesurée dans des conditions d'humidité relative de l'air de 95 % et l'air inclus dans l'emballage est réglé à une humidité relative de l'air entre 5 % et une valeur inférieure à 0,5 %.

2. Préparation selon la revendication 1, **caractérisée en ce que** l'air inclus dans l'emballage est réglé à une humidité relative de l'air entre 5 % et une valeur inférieure à 2 %.

3. Préparation selon la revendication 1, **caractérisée en ce que** l'air inclus dans l'emballage est réglé par addition d'un dessiccateur.

4. Préparation selon la revendication 1, **caractérisée en ce qu'**elle est disposée dans un système thérapeutique transdermique.

5. Préparation selon une ou plusieurs des revendications précédentes, **caractérisée en ce qu'**elle présente une couche adhérente dont le côté tourné vers la peau est auto-adhésif et une couche dorsale non-adhérente se détournant de la peau.

6. Préparation selon une ou plusieurs des revendications précédentes, **caractérisée en ce qu'**elle contient un copolymère d'ester acrylique.

7. Préparation selon la revendication 6, **caractérisée en ce que** le copolymère d'ester acrylique contient du 17-β-oestradiol sous forme dissoute à concurrence d'au moins 6 %, de préférence à concurrence d'au moins 8 % (g/g) dans des conditions d'humidité relative de l'air de 0,1 %.

8. Procédé pour la fabrication de préparations pharmaceutiques adhérant à la peau, soumises à un conditionnement primaire, selon une ou plusieurs des revendications précédentes, **caractérisé par** la préparation d'une solution ou d'une dispersion de semi-hydrate de 17-β-oestradiol dans un substrat pharmaceutique semi-solide ou liquide et par l'enduction ultérieure sur une matière de base en forme de feuille rendue anti-adhérente, par le séchage de la couche et par l'application d'une couche dorsale imperméable à la substance active et à l'humidité et par individualisation par estampage et par découpe en feuille, **caractérisé en ce que** le séchage a lieu par entreposage sous une humidité relative de l'air inférieure à 5 %, de préférence inférieure à 2 %, de manière particulièrement préférée inférieure à 0,5 %, jusqu'à la dissolution complète de l'oestradiol incorporé, et par conditionnement ultérieur dans un emballage hermétique étanche aux gaz, le cas échéant avec addition d'une matière absorbant l'humidité.

9. Utilisation de préparations selon une ou plusieurs des revendications 1 à 7 pour la fabrication d'une préparation pharmaceutique adhérant à la peau pour le traitement de symptômes liés à la ménopause, pour la prophylaxie de l'ostéoporose ou pour la prophylaxie et la thérapie de la maladie d'Alzheimer.
